# EUROPEAN PATENT APPLICATION

(11) **EP 1 946 790 A1**
(43) Date of publication of application: **23.07.2008**
(21) Application number: 08425001.8
(22) Date of filing: 02.01.2008
(51) Int. Cl.: A61M 21/00

(54) **Multisensorial diffuser with welness sequences related to meditation, sleep, relax and energy**

(30) Priority: 19.01.2007 IT MI20070069
(71) Applicant: N.P.S. Srl, 20099 Sesto San Giovanni (MI) (IT)
(72) Inventor: Gerosa, Ivan, 20099 Sesto San Giovanni Milan (IT)

(57) **Abstract**

The multisensorial diffuser is a programmable technique (a kind of light spot or lamp) able to distribute four reference therapies at the same time: aromatherapy, chromotherapy, musicotherapy and applied crystal therapy.

Innovation, compared to the current products made and of public access, is the synergetic union ("holistic") of these four therapies, with a product that can provide them at the same time by means of a special control to check the component groups and the specific fruition programmes.

## Description

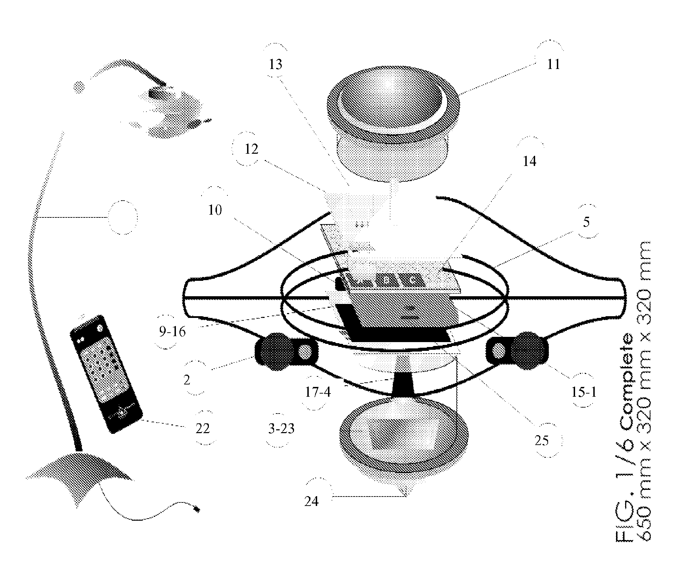

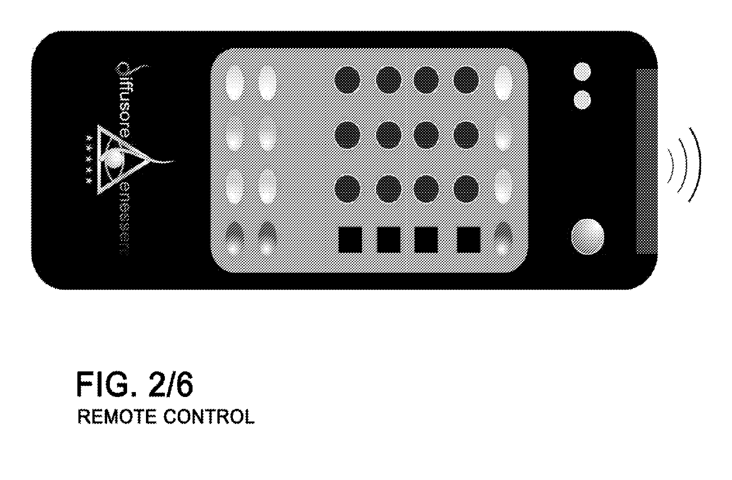

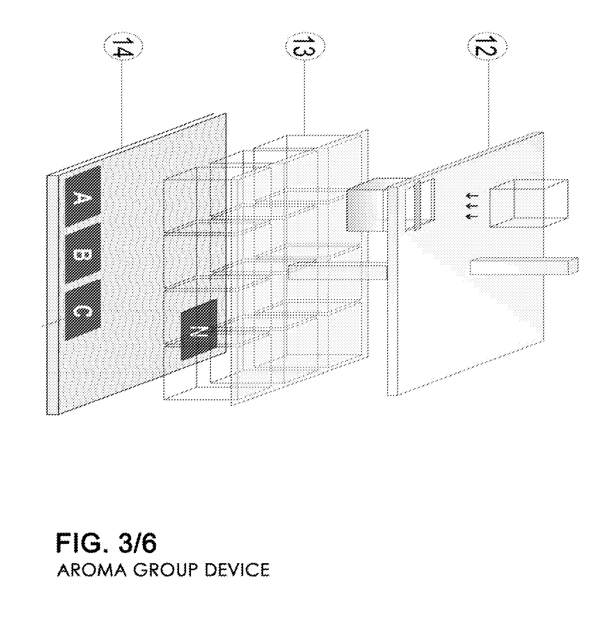

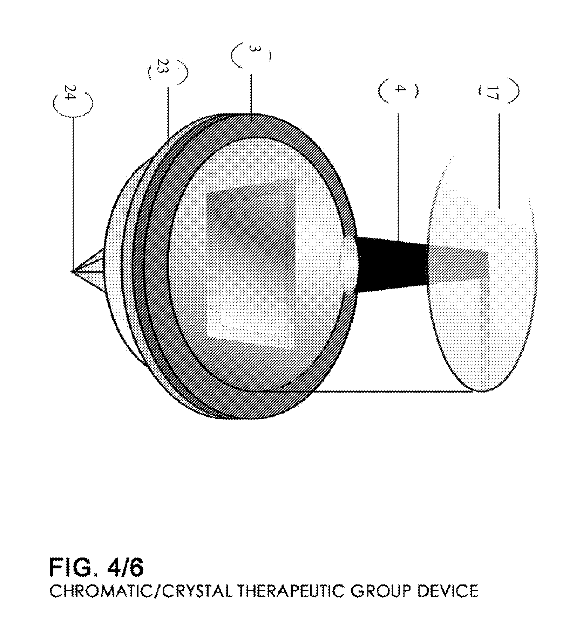

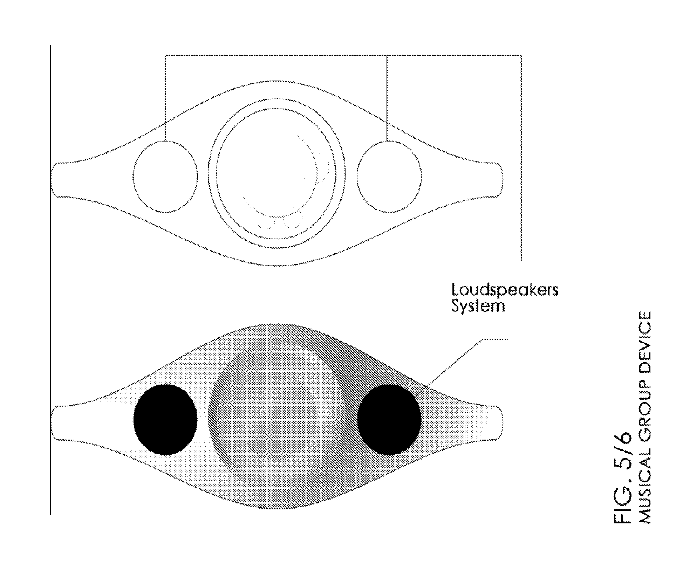

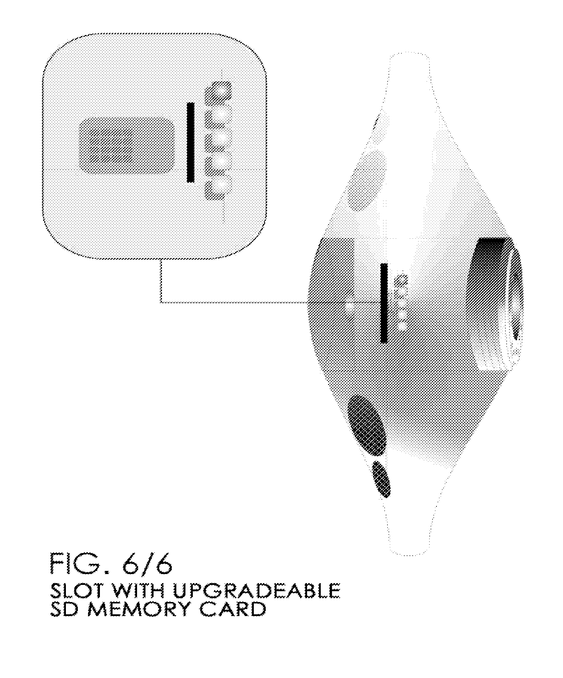

In the image "complete" (figure 1/6) are shown and indicated all the components of the multisensorial diffuser, with reference to the "components chart" summed up in succession. Such components, duly assembled in a sort of light spot able not only to distribuite the normal function of a neon lamp (component No. 5) by means of the remote control (component No. 5, figure 2/6), but also to check the activation of the aromas group (figure 3/6), of the chromatic/crystal therapeutic group (figure 4/6) and of the musical group (figure 5/6).

Each of these groups has been associated with sequences of aromas, colours, lights and musics specially carried out and selectable always by means of the special control (component No. 5, figure 2/6) according to the programmes:
a) relax
b) sleep
c) meditation
d) energy.

Each sequence as well as any other programming of the various functions related to the four reference therapies is also updateable and then modifiable with a flash card (component No. 7, figure 6/6).

### Here is the "components chart" of the diffuser:

Component code / Description
- 1: Fan
- 2: Loudspeaker
- 3: Fresnel convex policarbonate sepia lens

- 4: 16-colour RGD led lamp
- 5: Neon circular light
- 6: Power to electricity grid
- 7: 1-flash digital card
- 8: Power cables
- 9: Electronic card and programmed processor (=cpu) mp3 player and connections and sensor for component No. 22
- 10: Transformer stabilizer Structural components
- 11: No. 1 plate (cap) sup. transparent with grid
- 12: No. 1 plate for aroma containers (cubes or cylinders) and support for extraction
- 13: No. 12 cubes (or tubes) glass cables
- 14: No. 1 support with 12 restistances /heating cells
- 15: No. 1 support with a fan grid and special fan
- 16: No. 1 support for electronic card comp. 9
- 17: No. 1 optical group support for led bulb (comp. 4)
- 18: Parallelepipedal hollow tower with support feet. Summed up components.
- 19: Upper body shell
- 20: Lower body shell
- 21: Wall clamp and /or telescopic floor support with attack screws.
- 22: Remote control for diffuser functions and therapeutical groups.
- 23: Opal glass disc
- 24: Natural hyaline crystal pyramid for diffusion/refraction of led light and opalescent glass disc.
- 25: IRDA receiver with various led panel related to the state and control of consumptions.

## Claims

1. Application: it refers to a product with the features of a light spot or a lamp, able to provide four reference therapies at the same time: aromatherapy, chromotherapy, musicotherapy and applied crystal therapy, see "complete" (picture1/6).

2. Control: the application is controlled by a remote control that controls: the ignition of the neon light, the test on all the diffuser functions (ignition and master sequences), the volume control and a short-cut menu for the programme to activate (relax,sleep, energy and meditation).

3. Integrated application groups: the application is summed up in the electrical and mechanical components, see previous "components chart", and is distinguishable for the following application subgroups: aromas group (picture 3/6), the chromatic/crystal therapeutic (picture 4/6) and musical group (picture5/6). As for the aromas group, heating technology is claimed (through the platelets comp. 14) coupled to ventilation technology (component 1), also with the use of neutral substances (apolare-glycol carbon middle chain solvent and/or APV solvent), that can optimize the aeriform diffusion of the aromas used and neutralise the dispersion of the same at room temperature. As for the chromatic and musical group,we have used respectively sequences of colours and notes carried out in order to make as effective as possible the purposes of the application and the reference programmes.

4. In particular, in this chromatic group claim is shown the innovative way of led light diffusion with a specific mechanism of applied crystal therapy. Such a therapy is carried out by means of a couple of lens (a formal convex one and another composed of a properly opalized glass disc) able to diffuse and refract the light spectrum generated by the led lamp also through a natural hyaline quartz crystal (known as rock crystal) properly set in the middle of the opal glass disc and similar to a pyramid designed to reproduce the sizes of the golden Section (applied by the same pyramid of Cheope and for which the ratio between the base and the height is close to the absolute value of 1,618. Herewith we also claim a system of counters and leds able to indicate the number of complete heatings to which oils were subjected, in order to allow a timely replacement of these, as well as the integrative function of still or moving images reproduction (photo and /or video), using a miniature projector.

5. Updating: The application also has the feature of update. In fact every sequence as well as any other programming functions related to the four reference therapies can also be updated and then modified by updating a flash card, specially programmed (component No. 7 picture 6/6).
